(19) **Europäisches Patentamt**
**European Patent Office**
**Office européen des brevets**

(11) **EP 4 025 898 B1**

(12) **EUROPEAN PATENT SPECIFICATION**

(45) Date of publication and mention
of the grant of the patent:
**19.06.2024  Bulletin 2024/25**

(21) Application number: **20767995.2**

(22) Date of filing: **02.09.2020**

(51) International Patent Classification (IPC):
**G01N 21/552** *(2014.01)*       **G01N 21/35** *(2014.01)*
**G01N 33/50** *(2006.01)*       **G01N 33/574** *(2006.01)*

(52) Cooperative Patent Classification (CPC):
**G01N 33/57407; G01N 21/35; G01N 21/552;**
**G01N 33/5091; G01N 33/57426;** G01N 2021/3595;
G01N 2030/743; G01N 2800/7028

(86) International application number:
**PCT/EP2020/074391**

(87) International publication number:
**WO 2021/043787 (11.03.2021 Gazette 2021/10)**

(54) **DISCERNING BRAIN CANCER TYPE**

UNTERSCHEIDUNG DES HIRNTUMORTYPS

DISCERNEMENT DU TYPE DE CANCER DU CERVEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.09.2019  GB 201912578**

(43) Date of publication of application:
**13.07.2022  Bulletin 2022/28**

(73) Proprietor: **Dxcover Limited**
**Glasgow G1 1XW (GB)**

(72) Inventors:
• **BAKER, Matthew, J**
**Glasgow G1 1RD (GB)**
• **CAMERON, James**
**Glasgow G1 1RD (GB)**

(74) Representative: **Marks & Clerk LLP**
**15 Fetter Lane**
**London EC4A 1BW (GB)**

(56) References cited:
**WO-A1-2017/221027**

• **NAKAGAWA MASATAKA ET AL: "Machine learning based on multi-parametric magnetic resonance imaging to differentiate glioblastoma multiforme from primary cerebral nervous system lymphoma", EUROPEAN JOURNAL OF RADIOLOGY, ELSEVIER SCIENCE, NL, vol. 108, 14 September 2018 (2018-09-14), pages 147-154, XP085532221, ISSN: 0720-048X, DOI: 10.1016/J.EJRAD.2018.09.017**
• **BURY DANIELLE ET AL: "Spectral classification for diagnosis involving numerous pathologies in a complex clinical setting: A neuro-oncology example", SPECTROCHIMICA ACTA. PART A: MOLECULAR AND BIOMOLECULAR SPECTROSCOPY, ELSEVIER, AMSTERDAM, NL, vol. 206, 1 August 2018 (2018-08-01), pages 89-96, XP085488844, ISSN: 1386-1425, DOI: 10.1016/J.SAA.2018.07.078**
• **HANDS JAMES R ET AL: "Brain tumour differentiation: rapid stratified serum diagnostics via attenuated total reflection Fourier-transform infrared spectroscopy", JOURNAL OF NEURO-ONCOLOGY, KLUWER, BOSTON, US, vol. 127, no. 3, 13 February 2016 (2016-02-13), pages 463-472, XP035925552, ISSN: 0167-594X, DOI: 10.1007/S11060-016-2060-X [retrieved on 2016-02-13]**

- JAMES M. CAMERON ET AL: "Developing infrared spectroscopic detection for stratifying brain tumour patients: glioblastoma multiforme vs. lymphoma", ANALYST, vol. 144, no. 22, 7 October 2019 (2019-10-07), pages 6736-6750, XP55736901, UK ISSN: 0003-2654, DOI: 10.1039/C9AN01731C

**Description**

**FIELD**

The present invention relates to methods of classifying whether a subject suspected of having a brain tumour has a glioma or a lymphoma. The invention also relates to a diagnostic kit for classifying whether a subject suspected of having a brain tumour has a glioma or a lymphoma and a method of facilitating the selection of treatment for a subject suspected of having a brain tumour.

**BACKGROUND**

Brain tumour incidence rates have been increasing since the early 1990s, rising by 34% in the UK alone. Despite an improvement in patient survival, only 14% of patients survive 10 years or more after diagnosis, and the average reduction in life expectancy of 20 years is the highest of all other cancers. Rapid and timely diagnosis and determination of tumour type is crucial to expediting management and patient outcomes.

The symptoms most frequently associated with brain tumour are non-specific, such as headache, presenting a challenge for doctors in identifying which patients with these common symptoms are most likely to have a brain tumour, and should have expedited brain imaging. Consequently, patients often visit their general practitioner (GP) multiple times before diagnosis and for nearly two thirds of patients, diagnosis is in the emergency department once they deteriorate. Existing referral guidelines lack sensitivity and specificity, with as few as 1.6% of patients referred for urgent brain imaging from primary care having a brain tumour, suggesting many brain scans are unnecessary.

Brain tumours are diagnosed on magnetic resonance imaging (MRI) or computed tomography (CT) brain imaging, but there are many different types, depending on the underlying cell of origin. Crucially, it is not possible to identify the tumour type with certainty from imaging alone. Different brain tumour types, for example central nervous system (CNS) lymphoma and glioblastoma (GBM), can have a similar appearance on MRI, but very different therapy options. Additionally, the status of a glioma may have a bearing on the degree of surgery, which may be required. For example, attempted maximum safe surgical resection may be more justified in patients with *IDH1*-mutant gliomas, whilst a more limited resection may be more appropriate for *IDH1*-wildtype gliomas

While some brain tumour types are most commonly treated by surgery, other types are most commonly treated using radiotherapy and/or chemotherapy. In the absence of a test to clearly distinguish between the different types of brain tumour, physicians often resort to surgery as a first pass in the hope that this will determine the type of tumour, and, if the tumour is of a type that can be treated by surgery, remove the tumour. However, brain surgery involves serious risks including stroke or death. For patients who are found to have a type of tumour which is not commonly treated by surgery, this is an unnecessary exposure to risk. It also leads to a delay in commencing treatment, such as chemotherapy and/or radiotherapy, that will be more suitable.

In recent times, various biomarkers within the blood have been identified as useful indicators of particular diseases. For instance, cytokines, chemokines, and growth factors are cell-signalling proteins that mediate a range of physiological responses, and are associated with various diseases. Such molecules are generally detected by either bioassay or immunoassay, both of which can be time consuming given that often only one analyte may be analysed at a time.

More recently, analytical techniques based on vibrational spectroscopy have emerged in the field of disease diagnostics. Fourier-transform infrared spectroscopy (FTIR) in particular has become increasingly popular in medical research. In FTIR spectroscopy, biological samples are irradiated with infrared (IR) light. The absorbance of this light causes molecular vibrations and transitions within the sample, resulting in an IR spectrum which represents a biochemical fingerprint, and can characterise and quantify the levels of proteins, lipids, carbohydrates and nucleic acids that are present. The imbalances in these biomolecular components can give an indication of disease states.

There is a clear need for a method which can accurately and rapidly distinguish between brain tumour types without requiring brain surgery. The present invention aims to address one or more of the aforementioned issues.

Nakasawa et al, "Machine learning based on multi-parametric magnetic resonance imaging to differentiate glioblastoma multiforme from primary cerebral nervous system lymphoma", European Journal of Radiology, 2018, Volume 108p1-288, discloses an evaluation of the performance of a machine learning method based on texture features in multi-parametric magnetic resonance imaging (MRI) to differentiate a glioblastoma multiforme (GBM) from a primary cerebral nervous system lymphoma (PCNSL).

WO2017221027A1 (Baker et al) discloses a method of diagnosing and/or prognosing an abnormality, such as a proliferative disorder in a subject, which includes performing an IR spectroscopic analysis of a wet biofluid sample, such as a blood sample (or component thereof) from the subject; to produce a spectroscopic signature characteristic of the biofluid/blood sample (or component thereof).

**DESCRIPTION**

At its broadest, the present disclosure relates to the use of Attenuated Total Reflection FTIR on a sample from a subject in determining whether the subject has a glioma or a lymphoma.

According to a first aspect, there is provided a method of classifying whether a subject suspected of having a brain tumour has a glioma or a central nervous system lymphoma, according to claim 1.

Distinguishing between different brain tumour types, such as glioma and lymphoma is notoriously difficult. Current methods, which attempt to distinguish between the two, such as MRI, are often not accurate and are expensive to perform. Other methods, such as brain surgery are invasive to the patient and have a high level of risk. These methods are also time-consuming. This can delay the onset of treatment for the patient. Given the seriousness of brain tumours, time is of the essence; any unnecessary delay could affect the subject's chance of survival. The present invention provides a rapid test to distinguish between glioma and lymphoma in a patient. This enables a physician/clinician to quickly select and commence the most appropriate treatment for the subject, thereby reducing time delays and increasing the chance of the subject responding to treatment. In addition, the present method is non-invasive, relative to brain surgery, with only a blood sample from the subject required for analysis. Advantageously, this ensures that subjects are not unnecessarily exposed to the serious risks of brain surgery. The test also has a reasonably high degree of accuracy in distinguishing between a glioma and a lymphoma, as well as being cheaper than present methods to determine the brain tumour type.

In embodiments, determining whether the subject has a glioma or a lymphoma using the obtained spectroscopic signature characteristic of the blood sample (or component thereof) comprises analysing the obtained spectroscopic signature characteristic of the blood sample (or component thereof) to obtain an analysis which indicates whether the subject has a glioma or a lymphoma.

Analysis of the obtained spectroscopic signature characteristic of the blood sample (or component thereof) may comprise comparing the obtained spectroscopic signature characteristic of the blood sample (or component thereof) to one or more control spectroscopic signatures.

Advantageously, the analysis provides a classification of the subject into a subject having a lymphoma or a subject having a glioma. The analysis is typically presented to the clinician, in order to facilitate the clinician with their determination. This reduces the possibility of user error by the clinician, thereby improving the accuracy of determination and reducing the burden on the clinician.

The inventors have found that the obtained spectroscopic signature can be analysed by applying one or more algorithms to the obtained spectroscopic signature. Thus, in embodiments, analysis of the obtained spectroscopic signature characteristic of the blood sample (or component thereof) comprises applying an algorithm(s) to the obtained spectroscopic signature characteristic of the blood sample (or component thereof).

In some embodiments applying an algorithm(s) to the obtained spectroscopic signature characteristic of the blood sample (or component thereof) comprises or further comprises comparing the obtained spectroscopic signature characteristic of the blood sample (or component thereof) to one or more control spectroscopic signatures.

In embodiments, the control spectroscopic signature comprises one or more pre-correlated signatures previously determined to be from lymphoma and/or glioma subjects. The control spectroscopic signature may comprise a plurality of pre-correlated signatures stored in a database (e.g. a "training set") in order to derive a correlation with a determination of glioma or lymphoma. For example, the method may comprise applying an algorithm which uses the database or is at least partly developed from the database. One or more control spectroscopic signatures is described in more detail further below.

The application of one or more algorithms to the obtained spectroscopic signature enables the classification of the subject into a subject likely having a glioma or a subject likely having a lymphoma. This ability to determine whether the subject has a glioma or a lymphoma using the obtained spectroscopic signature characteristic of the blood sample (or component thereof), is especially surprising given that there are only small and/or few differences between the spectroscopic signature obtained from a subject having a glioma and the spectroscopic signature obtained from a subject having a lymphoma.

The algorithm may comprise a predictive model. The predictive model may be developed by "training" (e.g. via pattern recognition algorithms) a database of pre-correlated signatures. Thus, determining whether the subject has a glioma or a lymphoma using the obtained spectroscopic signature characteristic of the blood sample (or component thereof) may comprise correlating the obtained spectroscopic signature with a determination of glioma or lymphoma using a predictive model.

By "pre-correlated signature" this will be understood to refer to a signature already determined to correlate with a determination of glioma or lymphoma. For example, the pre-correlated signature may have been obtained from a blood sample (or component thereof) isolated from a subject known to have a lymphoma or glioma. Thus, in some embodiments, the method further comprises compiling a database of pre-correlated signatures by obtaining spectroscopic signatures from blood samples (or components thereof) isolated from subjects already known to have glioma or lymphoma.

Training a database of pre-correlated signatures may comprise applying a classification model to the pre-correlated spectroscopic signatures. An algorithm(s), for example a pattern recognition algorithm obtained using the classification model can then be applied to the obtained spectroscopic signature characteristic of the blood sample (or component thereof) to determine whether the subject has a glioma or a lymphoma.

Suitable classification models may include, but not be limited to random forest, support vector machine and partial least squares discriminant analysis. The inventors have found that each of these models is capable of determining whether the subject has a glioma or a lymphoma. In some embodiments the classification model comprises partial least squares discriminant analysis. Without wishing to be bound by theory, the present inventors believe partial least squares discriminant analysis is especially accurate at determining whether a subject has a glioma or a lymphoma. This is entirely unexpected to the present inventors.

In some embodiments the classification model further comprises one or more non-biasing methods. Non biasing methods are helpful to ensure that there is no bias when a training set, or set of samples, is biased in one direction, for example, a higher number of samples from subjects having a glioma versus subjects having a lymphoma.

Suitable non-biasing methods include, but are not limited to up-sampling, down-sampling and synthetic minority over-sampling technique (SMOTE). Up-sampling comprises repeatedly sampling the minority class to increase the number of samples (Simafore et al., 2019), whereas down-sampling selects a subset of the majority class at random, removing the extra samples to make it the same size as the minority class. SMOTE artificially mixes the data to create 'new' samples to achieve a more balanced dataset (Chawla et al., 2002).

In some embodiments, the classification model further comprises up-sampling or SMOTE. The classification model may further comprise SMOTE.

In some embodiments the classification model comprises SMOTE and one of random forest, support vector machine and partial least squares discriminant analysis.

In embodiments, the classification model comprises random forest and SMOTE, partial least squares discriminant analysis and SMOTE or support vector machine and up-sampling.

In some embodiments the predictive model, database of pre-correlated signatures and/or algorithm is provided.

In some embodiments, the methods described herein, may further comprise detecting a status of one or more biological markers in the blood sample, or component thereof. Detecting a status of one or more biological markers may be detecting whether or not said one or more markers comprises a mutation or mutations, or is present as wild type.

Detecting biomarker status may be carried out by any means known in the art, but the inventors have advantageously discovered that biomarker status may be determined using ATR-FITR. Thus, the present disclosure, in some embodiments, not only provides a method of discerning whether a subject has a glioma or lymphoma, using ATR-FITR on a blood sample, or a component thereof, but also a type or grade of glioma, based on biomarker(s) status using ATR-FITR.

In some embodiments, the method of detecting a status of one or more biological markers in the blood sample, or component thereof is conducted on subjects identified in accordance with the present disclosure, as having a glioma. Although, in some embodiments, the method of detecting a status of one or more biological markers in the blood sample, or component thereof may be carried out at the same time, or concurrently with detecting whether or not a subject has a glioma or a lymphoma.

The method of detecting a status of one or more biological markers in the blood sample, or component thereof, may be conducted on a size-fractionated sample obtained from the blood sample, or component thereof. Size-fractionation may permit biomolecules of different molecular weights to be separated, typically by centrifugation, into different fractions. For example, commercially available ultra-centrifugal filtering devices (see Amicon Ultra, for example), allow samples to be centrifuged, such that higher molecular weight material is held within a concentrate, with lower molecular weight material passing through a filter and maintained within a filtrate. Centrifugal filtering devices are available with differing molecular weight cut-off values, in order to separate higher molecular weight material from lower molecular weight material. In order to separate specific biomarkers from higher molecular weight material, filtering devices, which can filter material of less than 20, 15, 10, 5, or 3kDa may be employed.

In one embodiment, the marker is isocitrate dehydrogenase 1 (IDH1). Somatic mutations in the human cytosolic isocitrate dehydrogenase 1 (*IDH1*) gene is a frequent feature observed in gliomas. The *IDH1* mutation tends to occur in the early stages of gliomagenesis. It is most commonly found in the low-grade gliomas, diffuse astrocytoma and oligodendroglioma, but is less common (10%) in the malignant glioma, glioblastoma (GBM), except where the GBM develops from a previously diagnosed diffuse or anaplastic astrocytoma (>80%). Consequently, the *IDH1* mutation serves as a valuable diagnostic marker, by assisting in the differentiation of tumour entities that are often indistinguishable through histopathological analysis alone, but have different treatments and prognostic profiles.

As well as IDH1, other biomarkers are known to be associated with different grades or types of glioma and as such detecting the status of such biomarkers, may permit further differentiation on the type of glioma a subject may have. The table below identifies, common genetic and chromosomal aberrations associated with the major glioma subtypes.

| Glioma entity | WHO grade | IDH1 mutation | Additional associated molecular alterations |
|---|---|---|---|
| Pilocytic astrocytoma | I | Extremely rare | BRAF, KRAS, NF1, FGFR1 |
| Diffuse astrocytoma | II | Common | IDH2, TP53, ATRX, LOH 17p |
| Anaplastic astrocytoma | III | Common | IDH2, TP53, ATRX, LOH 17p |
| Oligodendroglioma | II | Majority of cases | IDH2, TP53, ATRX, 1p/19q codeletion |
| Anaplastic oligodendroglioma | III | Majority of cases | IDH2, TP53, ATRX, 1p/19q codeletion |
| Glioblastoma (primary) | IV | Rare | TERT, PTEN, TP53, MGMT, EGFR |
| Glioblastoma (secondary) | IV | Extremely Common | IDH2, TP53, ATRX, LOH 17p |

NF1, neurofibromatosis type 1; FGFR1, fibroblast growth receptor 1; IDH2, isocitrate dehydrogenase 2; TP53, tumour suppressor protein 53; ATRX, alpha thalassemia/mental retardation syndrome X-linked mutation; LOH 17p, loss of heterozygosity on chromosome 17; TERT, telomerase reverse transcriptase; PTEN, phosphatase and tensin homolog; MGMT, O(6)-methlyguanine-DNA-methyltransferase; EGFR, epidermal growth factor receptor.

Understanding a subject's biological marker status (e.g. IDH1 status) can give an indication of prognosis and therefore can provide information to a surgeon in terms of how to limit the extent of resection/how aggressive they are with surgery, e.g. attempted maximum safe surgical resection may be more justified in patients with IDH1-mutant gliomas, whilst a more limited resection may be more appropriate for IDH1-wildtype gliomas.

As used herein, "component thereof" of a blood sample refers to a component of a blood sample such as plasma or serum. Herein, "plasma" refers to the straw-coloured/pale-yellow liquid component of blood that normally holds the blood cells in whole blood in suspension. It makes up about 55% of total blood volume. It is the intravascular fluid part of extracellular fluid (all body fluid outside of cells). It is mostly water (93% by volume) and contains dissolved proteins (major proteins are fibrinogens, globulins and albumins), glucose, clotting factors, mineral ions (Na+, Ca", Mg", HCO3 Cl- etc.), hormones and carbon dioxide (plasma being the main medium for excretory product transportation). It is to be noted that, for plasma samples, both EDTA plasma and citrate plasma are suitable. Heparin plasma is also suitable. In the context of the present invention, "serum" refers to the component that is neither a blood cell (serum does not contain white or red blood cells) nor a clotting factor; it is the blood plasma with the fibrinogens removed.

Conventionally, an ATR-FTIR spectrometer has a point of analysis, known as an internal reflection element (IRE). During spectroscopic analysis, a beam of infrared light is passed through the IRE, on which the sample is supported. The beam is internally reflected in the IRE, forming an evanescent wave at the IRE-sample interface. This evanescent wave interrogates the sample at a defined penetration depth. As the beam or "evanescent wave" exits the IRE, the beam or evanescent wave is received by an infrared detector. Each interrogation of the sample by an evanescent wave may otherwise be referred to as a scan.

Thus, it will be understood that "spectroscopic analysis" may be otherwise referred to herein as infrared analysis.

Spectroscopic analysis may comprise at least one scan of the blood sample (or a component thereof). In some embodiments, the spectroscopic analysis comprises a plurality of scans of the blood sample (or a component thereof). A plurality of scans may comprise at least 2, at least 4, at least 6, at least 8, at least 10, at least 12, at least 14, at least 16, at least 18 or at least 20 scans of the blood sample (or a component thereof). In some embodiments, spectroscopic analysis comprises at least 30 scans of the blood sample (or a component thereof), at least 40 scans, at least 50 scans, and at least 100 scans. In embodiments, spectroscopic analysis comprises at least 2 scans scans and no more than 100 scans, optionally at least 30 scans and no more than 100 scans. In embodiments spectroscopic analysis comprises at least 10 scans and no more than 40 scans, optionally at least 10 scans and no more than 30 scans. In some embodiments, spectroscopic analysis comprises 16 scans. In embodiments spectroscopic analysis comprises 32 scans. The number of scans is suitably selected to optimize data content and data-acquisition time.

In the context of the present invention, the term "spectroscopic signature" is used to refer to the infrared spectrum obtained from a blood sample (or component thereof). The infrared spectrum can be visualised in a graph to show infrared light absorbance or emittance. Thus, in embodiments, the obtained spectroscopic signature refers to the infrared spectrum of a blood sample (or component thereof) visualised in a graph to show infrared light absorbance.

As the skilled person will appreciate, the level of absorbance of sections of the infrared spectrum can be used to indicate the type and proportion of molecule present in the blood sample (or component thereof). This is because each molecule in the blood sample (or component thereof) has one or more vibrational frequencies; when the frequency of the infrared radiation matches the vibrational frequency, absorbance occurs. Typical units of frequency of infrared

radiation are cm$^{-1}$.

In embodiments, the spectroscopic signature characteristic of the blood sample (or component thereof) is the spectrum between 4000 and 400 cm$^{-1}$. By spectrum between 4000 and 400 cm$^{-1}$, this will be understood to refer to the infrared spectrum obtained at between 4000 and 400 cm$^{-1}$ of infrared radiation.

In embodiments the spectroscopic signature characteristic of the blood sample (or component thereof) is the spectrum between 3000 and 500 cm$^{-1}$. Optionally, the spectroscopic signature characteristic of the blood sample (or component thereof) is the spectrum between 2000 and 600 cm$^{-1}$, optionally 900 and 1800 cm$^{-1}$. By spectrum between 900 and 1800 cm$^{-1}$, this will be understood to refer to the infrared spectrum obtained at between 900 and 1800 cm$^{-1}$ of infrared radiation.

Without wishing to be bound by theory, the present inventors believe that the spectrum between 900 and 1800 cm$^{-1}$ indicates the type and proportion of molecules which differ between a subject having a glioma and a subject having a lymphoma. Although these differences may be minor, the inventors have advantageously found that further analysis of the obtained spectroscopic signature can be used to determine whether the subject has a glioma or a lymphoma based on these differences.

In some embodiments, the spectroscopic signature characteristic of the blood sample is the spectrum between 900, 1000, 1100, 1200, 1300 or 1400 and 1500, 1600, 1700 or 1800 cm$^{-1}$. In some embodiments the spectroscopic signature characteristic of the blood sample (or component thereof) is the spectrum between 1400 and 1800 cm$^{-1}$. The spectroscopic signature characteristic of the blood sample (or component thereof) may be the spectrum between 1500 and 1700 cm$^{-1}$, optionally between 1550 and 1700 cm$^{-1}$. The spectrum between 1500 and 1700 cm$^{-1}$ will be understood to comprise the Amide I and Amide II regions. The Amide I and Amide II regions are regions of the spectrum which are known in the art to relate to the absorbance or emittance of proteins in the blood sample (or component thereof) (Barth *et al.* and Glassford *et al.*). The present inventors have discovered that the Amide I and/or Amide II region contain differences between a subject having a glioma and a subject having a lymphoma. Thus, the Amide I and/or Amide II region can advantageously be analysed, for example, using one or more algorithms, to determine whether the subject has a glioma or a lymphoma.

The spectrum between 1150 and 1000 cm$^{-1}$ relates to the absorbance or emittance of nucleic acid material, glycogen and carbohydrates.

The spectroscopic signature may comprise or consist of a portion of the spectrum obtained from the blood sample. Thus, in embodiments, the spectrum (or spectra) obtained from the blood sample (or component thereof) may be in the range 5000-100 cm$^{-1}$, 4000 - 400 cm$^{-1}$ or 4000 - 450 cm$^{-1}$. The spectroscopic signature may then comprise a portion of this obtained range, for example the spectrum between 900, 1000, 1100, 1200, 1300 or 1400 and 1500, 1600, 1700 or 1800 cm$^{-1}$. The spectroscopic signature effectively acts as a biochemical fingerprint for the subject.

The spectrum (or spectra) may have a resolution of 10 cm$^{-1}$ or less, 5 cm$^{-1}$ or less, or 4 cm$^{-1}$ or less. In embodiments, the spectrum (or spectra) may have a resolution of 1 to 4 cm$^{-1}$.

In embodiments, "ATR crystals" support the blood sample (or component thereof) during spectroscopic analysis. ATR crystals are fixed IREs. The ATR crystals may be formed of diamond, zinc selenide or germanium. In embodiments, the ATR crystals comprise or consist of a single reflection diamond crystal.

In embodiments a silicon internal reflection element (IRE) supports the blood sample (or component thereof) during spectroscopic analysis. Silicon IREs are cheaper than ATR crystals. Conveniently, silicon IREs are disposable (and so not fixed like ATR crystals), thereby enabling high-throughput analysis of multiple sampling points. Silicon IREs also enable batch-analysis, and the option of repeating analysis if required.

The blood sample may have a volume of about 0.1 to 10μl, optionally a volume of about 0.1 to 5μl. In some embodiments the blood sample comprises a volume of about 3μl.

The method may further comprise applying the blood sample (or component thereof) to the surface of the ATR crystal or silicon IRE prior to spectroscopic analysis. Once applied to the surface of the ATR crystal or silicon IRE, the blood sample may be dried for a period of time prior to spectroscopic analysis, optionally between 5 minutes and 2 hours. In some embodiments the blood sample may be dried for between 30 minutes and 90 minutes, optionally one hour, prior to spectroscopic analysis. Drying may be carried out at a temperature of at least 20°C and no more than 40°C, preferably at a temperature of at least 30°C and no more than 37°C.

Once the blood sample has dried on the surface of the ATR crystal or silicon IRE, it may be otherwise referred to as a blood sample film. The blood sample film may be of a substantially uniform thickness within a tolerance of +/- 40μm or less.

The average film thickness of the blood sample (or component thereof) across the surface of the ATR crystal or silicon IRE (or at least the part of it exposed to spectroscopic analysis) may be between 0.1 and 200 μm, optionally between 1 and 100 μm, optionally between 2 and 50 μm. The maximum film thickness (i.e. the point of maximum thickness) of the blood sample (or component thereof) across the surface of the ATR crystal or silicon IRE (or at least the part of it exposed to spectroscopic analysis) may be between 1 and 200 μm, optionally between 2 and 100 μm. In embodiments the maximum film thickness is between 5 and 50 μm, optionally between 2 and 8 μm. The minimum film

thickness (i.e. the point of minimal thickness) of the blood sample (or component thereof) across the surface of the ATR crystal or silicon IRE (or at least the part of it exposed to spectroscopic analysis) may be between 0 and 40 $\mu$m, optionally between 1 and 20 $\mu$m, further optionally between 2 and 10 $\mu$m.

Analysis of the resulting film via White Light Interferometry can indicate the thickness of the film across the surface of the ATR crystal or silicon IRE, so as to verify the appropriate film thickness. The inventors have found that producing films of the appropriate thickness can reduce signature variance associated with sample preparation, such that any observed variance in signatures from blood sample to blood sample can be more reliably attributed to differential compositions rather than variability in sample preparation.

In embodiments, the blood sample (or component thereof) comprises a portion of a bulk blood sample isolated from the subject. In this manner, further portions of the bulk blood sample can be later used for further spectroscopic analyses, thereby assisting validation of results. At least two spectroscopic analyses may be performed on each blood sample, optionally at least three. Optionally, each individual spectroscopic analysis is repeated at least twice with the same sample, preferably at least three times, to help validate results.

Classifying whether the subject has a glioma or a lymphoma using the obtained spectroscopic signature characteristic of the blood sample (or component thereof) may be carried out by a clinician. In embodiments, determining whether the subject has a glioma or a lymphoma using the obtained spectroscopic signature characteristic of the blood sample (or component thereof), is automated. Automation may be computational, for example by computer software installed on a computer or on a medium for use by a computer.

In embodiments, the application of one or more algorithms to the obtained spectroscopic signature is automated. For example, the application of one or more algorithms to the obtained spectroscopic signature may be by computer software. The computer software may be installed on a computer or on a medium for use by a computer.

The inventors have surprisingly found that at least a portion of the obtained spectroscopic signature differs depending on whether the subject has a glioma or a lymphoma. This is due to different light absorption in samples from a subject having a glioma versus a subject having a lymphoma. Thus, in embodiments wherein the obtained spectroscopic signature(s) refers to the infrared spectrum of a blood sample (or component thereof) displayed in a graph to show infrared light absorbance, at least a portion of the obtained spectroscopic signature characteristic of the blood sample (or component thereof) of a subject with glioma may display a lower infrared light absorbance than a corresponding portion of the obtained spectroscopic signature characteristic of the blood sample (or component thereof) of a subject with lymphoma. Likewise, at least a portion of the obtained spectroscopic signature characteristic of the blood sample (or component thereof) of a subject with lymphoma may display a higher infrared light absorbance than a corresponding portion of the obtained spectroscopic signature characteristic of the blood sample (or component thereof) of a subject with glioma. The at least a portion of the obtained spectroscopic signature may refer to the spectrum between 1400 and 1800 cm$^{-1}$, optionally the spectrum between 1500 and 1700 cm$^{-1}$, or optionally the spectrum between 1550 and 1700 cm$^{-1}$.

The portion of the obtained spectroscopic signature may comprise or consist of the obtained spectrum from the blood sample (or component thereof) between 1400 and 1800 cm$^{-1}$, optionally the obtained spectrum from the blood sample (or component thereof) between 1500 and 1700 cm$^{-1}$, or between 1550 and 1700 cm$^{-1}$.

For example, when the portion of the obtained spectroscopic signature comprises or consists of the obtained spectrum from the blood sample (or component thereof) between 1400 and 1800 cm$^{-1}$, the "corresponding portion" of the obtained spectroscopic signature will be understood to comprise or consist of the spectrum between 1400 and 1800 cm$^{-1}$.

In embodiments, the subject is determined to have the glioma when at least a portion of the obtained spectroscopic signature characteristic of the blood sample (or component thereof) is lower than at least a portion of a control spectroscopic signature. The subject may be determined to have the lymphoma when at least a portion of the obtained spectroscopic signature characteristic of the blood sample (or component thereof) is higher than at least a portion of a control spectroscopic signature. In such embodiments, it will be appreciated that the spectroscopic signature obtained from the blood sample and the control spectroscopic signature will both relate to infrared light absorbance, or both relate to infrared light emittance, such that comparisons can be made.

The obtained spectroscopic signature may be pre-processed prior to determining whether the subject has a glioma or a lymphoma using the obtained pre-processing spectroscopic signature characteristic of the blood sample (or component thereof).

Pre-processing techniques are well known to those skilled in the art. Pre-processing may include one or more of normalisation of the obtained spectroscopic signature, baseline correction of the obtained spectroscopic signature, data reduction of the obtained spectroscopic signature and/or binning of the obtained spectroscopic signature. Normalisation may comprise normalising the obtained spectroscopic signature relative to one or more spectroscopic signatures from a healthy subject or subject known to not have a brain tumour.

Pre-processing may be carried out using computer software installed on a computer. In embodiments the pre-processing computer software comprises the computer software RStudio. The pre-processing may be carried out using the PRFFECT toolbox in RStudio computer software. Advantageously, pre-processing reduces unwanted variance in spectra.

Binning may be at a factor of 2, 4, 6, 8 or 10. In embodiments binning is at a factor of 8.

As used herein, the term lymphoma defines a cancer of the lymphocytes. The initiation of lymphoma generally occurs in the lymph nodes and/or organs of the lymphatic system. The lymphoma is central nervous system (CNS) lymphoma, optionally primary CNS lymphoma or secondary CNS lymphoma. The lymphoma may be in the brain.

The three main types of malignant glioma are astrocytomas, ependymomas and oligodendrogliomas. The present invention may relate to one or more of these types of glioma. A tumour with a mixture of the histological features present in the main three types of glioma is known as a mixed glioma, which the present invention may also serve to distinguish from a lymphoma. Table 1 below shows the sub-types of high grade and low-grade gliomas.

Table 1 Grading of Brain Tumours

| General Tumour Grade | WHO Grade | Grade Sub-type |
|---|---|---|
| Low Grade | I | Pilocytic astrocytoma |
| | II | Oligodendroglioma |
| | II | Astrocytoma |
| High Grade | III | Anaplastic astrocytomas |
| | III | Oligodendrogliomas |
| | IV | Glioblastoma multiforme |

In embodiments, the glioma is a low grade glioma. Alternatively, the glioma may be a high grade glioma. The glioma may comprise one or more of Pilocytic astrocytoma, Oligodendroglioma, Astrocytoma, Anaplastic astrocytomas, Oligodendrogliomas or Glioblastoma multiforme glioma sub-types.

In embodiments, the glioma is a Grade III or Grade IV glioma. The glioma may be a glioblastoma multiforme.

The subject may be suspected of having a brain tumour due to images previously taken of the subject's brain, for example Magnetic Resonance Images (MRI). In some embodiments the method comprises a preliminary step of imaging the subject's brain, the imaging optionally comprising using an MRI scanner to image the subject's brain.

Symptoms of a brain tumour may include, but not necessarily be limited to one or more of headache, nausea and/or vomiting, confusion, memory loss, personality change, difficult with balance, urinary incontinence, loss of vision, speech difficulties and seizures. Thus, the subject may be suspected of having a brain tumour if they present with one or more of the above symptoms.

The subject is an animal, preferably a mammalian animal. Mammalian animals include, but are not limited to horses, dogs, cats, birds, and humans. In some embodiments, the subject is a human subject.

In embodiments the blood sample is blood serum or blood plasma. In some embodiments, the blood sample is blood serum.

In embodiments, the blood serum is whole serum, most preferably whole human serum. Whole serum may be used directly in the spectroscopic analysis. Alternatively, the serum sample may be diluted according to the requirements of the spectroscope (e.g. sensitivity) and the homogeneity required of the sample being analysed.

In other embodiments, the blood serum is centrifugally filtered serum which has molecules above a certain molecular weight removed therefrom. For instance, the blood serum may be centrifugally filtered to remove components having a molecular weight above 100kDa (kilodaltons). In other embodiments, the blood serum may be centrifugally filtered to remove components having a molecular weight above 10kDa. In other embodiments, the blood serum may be centrifugally filtered to remove component having a molecular weight above 3kDa. Any or all of the abovementioned centrifugally filtered serums may be used directly in spectroscopic analysis. Alternatively, the centrifugally filtered serum sample may be diluted according to the requirements of the spectroscope (e.g. sensitivity) and the homogeneity required of the sample being analysed.

Where the blood sample is blood serum, the serum sample is suitably prepared by allowing an extracted blood sample to first clot, suitably at room temperature, suitably for between 25 minutes and 1 h 10 minutes. The serum is then suitably centrifuged or filtered to clear the sample of precipitate. Centrifuging is suitably performed at between 9000 and 20000 rpm, suitably between 10000 and 15000 rpm, suitably for 5-20 mins, suitably at 2-8°C. Filtering of serum samples suitably involves filtering through a 0.8/0.22 pm dual filter to prevent instrument clogging. The blood serum should then be either assayed immediately or otherwise aliquot and store serum samples in single use aliquots at -70°C. Before assaying, suitably the serum sample is diluted with an appropriate sample diluents. Suitably 1 volume of serum sample may diluted with 2-5 volumes of sample diluents, suitably with 3 volumes of sample diluents. The serum may be diluted 1:50 or 1:100.

In some embodiments, determining whether the subject has the glioma or the lymphoma using the obtained spectroscopic signature characteristic of the blood sample (or component thereof) facilitates a determination of treatment of the subject. For example, if the subject is determined to have the glioma this may facilitate the determination of at least one treatment of the subject and if the subject is determined to have the lymphoma this may facilitate the determination of at least one other different treatment of the subject. Common treatments for lymphoma and glioma vary. For example, if a subject is suspected of having a glioma, treatment by brain surgery, to remove the tumour, may be selected. In contrast, if a subject is suspected of having a lymphoma, non-surgical interventions such as chemotherapy and/or radiotherapy are considered to be more suitable. The present method thereby enables a physician to quickly and accurately select an appropriate treatment for the subject. The quick and accurate selection of an appropriate treatment improves the subject's chance of recovery. It also prevents unnecessary brain surgery in instances where the subject is determined to have lymphoma.

In embodiments, treatment with chemotherapy and/or radiotherapy is selected if the subject is determined to have the lymphoma and treatment with surgery is selected if the subject is determined to have the glioma. By surgery, this will be understood to refer to brain surgery with the aim of removing the glioma from the subject's brain.

According to a second aspect, there is provided a diagnostic kit for classifying whether a subject suspected of having a brain tumour has a glioma or a central nervous system lymphoma, according to the appended claims.

In embodiments, the device for performing spectroscopic analysis upon the blood sample (or component thereof) is the same as the device to determine whether the subject has a glioma or a central nervous system lymphoma.

In embodiments, the device configured to receive a blood sample comprises ATR crystals. In other embodiments, the device configured to receive a blood sample comprises a silicon IRE.

The device configured to receive a blood sample may be configured to automatically prepare a blood sample (or component thereof) of a required thickness and dryness.

The kit may comprise a centrifugal filter device to permit the separation of higher molecular weight material from lower molecular weight material, as further described herein.

According to a third aspect there is provided a computer-readable medium comprising computer software, according to the appended claims.

The present invention may be used in a method of facilitating the selection of treatment for a subject suspected of having a brain tumour. The method comprises performing spectroscopic analysis upon a blood sample (or component thereof) isolated from the subject to obtain a spectroscopic signature characteristic of the blood sample (or component thereof). The spectroscopic analysis is Attenuated Total Reflection FTIR (ATR-FTIR). The method further comprises determining whether the subject has a glioma or a central nervous system lymphoma using the obtained spectroscopic signature characteristic of the blood sample (or component thereof); and selecting a treatment based on the determination of glioma or lymphoma. Methods of treatment per se are not within the scope of the claims.

Treatment with chemotherapy and/or radiotherapy may be selected if the subject is determined to have the lymphoma and treatment with surgery may be selected if the subject is determined to have the glioma.

The method may further comprise administering the selected treatment to the subject. For example, if the subject is determined to have the central nervous system lymphoma, the subject may be treated with chemotherapy and/or radiotherapy. If the subject is determined to have the glioma, the subject may be treated with brain surgery.

All of the features described herein (including any accompanying claims, abstract and drawings) may be combined with any of the above aspects in any combination, unless otherwise indicated.

## DETAILED DESCRIPTION

Embodiments of the invention will now be described by way of example, and with reference to the accompanying figures, which show:

Figure 1 shows a pre-processing example; (a) raw data, and (b) pre-processed;

Figure 2 shows a Gini importance plot from RF analysis showing the mean spectra from lymphoma (black) and glioblastoma (red). Blue: Protein; Yellow: Lipid; Green: Nucleic acid and Orange: Carbohydrate;

Figure 3 shows PLS scores plot for Lymphoma (black) vs GBM (red);

Figure 4 shows loadings plot for the 2nd PLS component in the lymphoma vs GBM classification with added biological assignments;

Figure 5 shows bootstrapping analysis to determine sufficient number of resamples required for the lymphoma vs GBM patient dataset: (a) the sensitivity and (b) specificity; and

Figure 6 shows ROC curve displaying trade-off between sensitivity and specificity of the SVM + up-sampling classification of the lymphoma vs GBM patients.

Figure 7 shows examples of whole serum (bottom), the HMW concentrate (middle) and the LMW filtrate (top) spectra. Raw spectra offset for clarity.

Figure 8 shows single model receiver operator characteristic (ROC) graphs for the a) whole serum dataset displaying the PLS-DA (blue), SVM (red) and RF (green) classifiers; and b) the best performing model for each of the tested filtrate fractions: the full spectrum (4000-800 cm$^{-1}$, blue), the fingerprint region (1800-1000 cm$^{-1}$, red) and the extended fingerprint region (1800-800 cm$^{-1}$, green).

Figure 9 shows a) the PLS scores plot between PLS1 and PLS2 for the *IDH1*-mutated (black) and *IDH1*-wildtype (red) <3kDa serum filtrate (4000-800 cm$^{-1}$) dataset, and b) the loadings for the 2$^{nd}$ PLS component.

## Example 1 Lymphoma versus Glioblastoma

### Introduction

Neurologists are particularly interested in the differentiation of primary central nervous system (CNS) lymphoma from the highly aggressive stage IV tumour, glioblastoma multiforme (GBM). A serum diagnosis would be beneficial for two reasons; firstly, it can often be difficult to distinguish between them through brain scans, such as magnetic resonance imaging (MRI), and secondly, it determines whether the tumour will be surgically removed or not. If an MRI scan suggests a patient has GBM, then they will be urgently sent for a resection. On the other hand, if it is thought that the tumour is lymphoma, they do not immediately operate on the patient, and the patients are treated with chemo- and radiotherapy. The ambiguity arising from brain scans make it extremely difficult for neurologists to effectively decide on the best course of action.

### Methods

#### Sample collection and preparation

Serum samples were obtained from three sources; the Walton Centre NHS Trust (Liverpool, UK), Royal Preston Hospital (Preston, UK), and the commercial source Tissue Solutions Ltd (Glasgow, UK). The number of serum samples obtained from each source is shown in Table 2. Ethical approval for this study was obtained (Walton Research Bank and BTNW/WRTB 13_01/ BTNW Application #1108).

**Table 2 - Serum samples used for the Lymphoma vs GBM differentiation**

|  | GBM | Lymphoma |
|---|---|---|
| Liverpool | 46 | 23 |
| Preston | 25 | 18 |
| **Total** | **71** | **41** |

In order to be included in this study, the cancer patients must have had a pathologically confirmed primary lymphoma or glioblastoma brain tumour, and must not have been undergoing chemo- or radio-therapy at the time of collection. Blood samples were collected in serum collection tubes and allowed to clot for up to one hour. The tubes were centrifuged at 2200g for 15 minutes at room temperature, then the separated serum component was subsequently aliquoted and stored in an -80°C freezer.

Prior to spectral analysis, the frozen serum samples were removed from storage and thawed at room temperature (18-25°C) for an average time of 15-20 minutes. Using a micropipette, 3$\mu$L of serum from one individual patient was deposited onto each of the three sample wells of the optical sample slide (wells 1, 2 and 3), whilst ensuring well '0' remained clean for background collection (ClinSpec Diagnostics Ltd, UK). The serum drops were spread across the well using the pipette tip, in order to create a thin serum film and cover the whole IRE for more uniform deposition. Prepared slides were stacked in 3D printed polylactic acid (PLA) slide holders, which were designed to enable batch drying. The stacked slides were then stored in a drying unit incubator (Thermo Fisher™ Heratherm™, GE) at 35°C for 1 hour. This step provides even heat and airflow for controlled drying dynamics of the serum droplet, to obtain a smooth, flat homogenous sampling surface.

Spectral collection

For this study, a Perkin Elmer Spectrum 2 FTIR spectrometer (Perkin Elmer, UK) was used for the spectral collection. A Specac Quest ATR accessory unit was fitted with a specular reflectance puck (Specac Ltd, UK), allowing the SIRE (silicon IRE) to sit on top of the aperture and replace the traditional fixed diamond IRE. The Slide Indexing Unit (ClinSpec Diagnostic Ltd, UK) enabled accurate and reproducible movement across the specular reflectance puck, indexing the optical slide between sample wells. With the first well acting as a background, the three sample wells provide the biological repeats. Each well was analysed in triplicate - resulting in nine spectra per patient. The spectra were acquired in the range 4000-450cm$^{-1}$, at a resolution of 4cm$^{-1}$, with 1cm$^{-1}$ data spacing and 16 co-added scans.

Spectral pre-processing

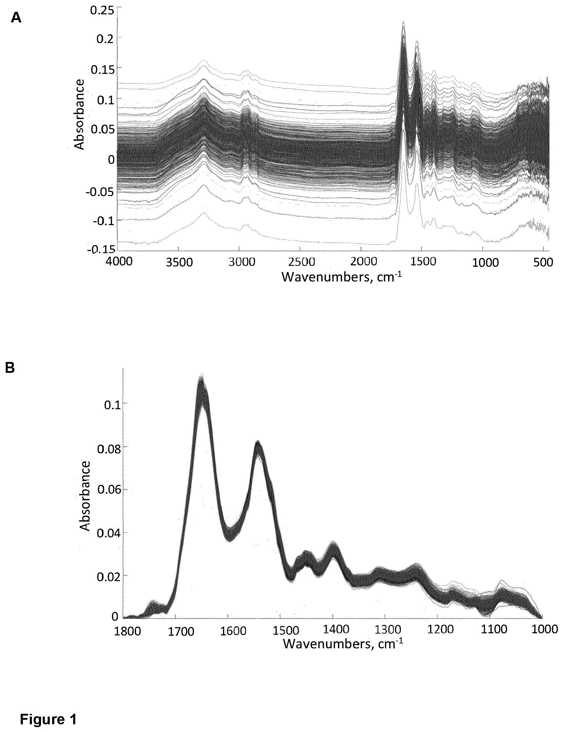

Here we have used the PRFFECT toolbox within RStudio software for the spectroscopic analysis, which can be divided into two parts; spectral pre-processing and spectral classification. The pre-processing step is commonly applied in spectroscopic studies, as it reduces unwanted variance in the dataset. A combination of baseline correction, normalisation and data reduction enables the significant biological information be emphasised and improves the classification performance. The optimum pre-processing protocol was determined using a trial-and-error iterative approach. The PRFFECT toolbox offers various pre-processing methods, such as binning, smoothing, normalisation and numerical derivatives - we direct the reader towards Smith *et al* (2). for more information on the use of this open-source program. Figure 1 gives an example of data pre-processing; (a) is the mean plot for a whole unprocessed dataset, and (b) shows the spectra cut to a fingerprint region (spectroscopic signature), with baseline correction and a vector normalisation applied - greatly reducing the spectral variation.

The optimal pre-processing parameters were found to be (in order); extended multiplicative signal correction (EMSC), spectral cut to the fingerprint region (1800-1000cm$^{-1}$), a minmax normalisation and a binning factor of 8.

Spectral analysis

The classification step consists of the actual disease predictions; the purpose of this approach is to identify the biosignature from a known patient cohort to develop a trained classification model, and then to use this information to predict the presence of disease in an unknown population.

To train the classification models, patients were randomly split into training and test sets, with a 70:30 split. In order to make the predictions more robust, no single patient could appear in more than one of these portions. Models were tuned on the training set (70%) and then used to make predictions for the spectra in the test set (30%), whilst employing a 5-fold k-cross validation. The consensus vote amongst the nine spectra that were analysed for each patient was reported as the diagnostic outcome (GBM or Lymphoma).

Model performance is reported in terms of sensitivity, specificity, kappa and balanced accuracy. Sensitivities and specificities (Eq. 1 and 2), are based on the number of correct and incorrect predictions in the external test set. The sensitivity generally refers to the ability of a test to correctly identify the patients with disease and the specificity tends to describe the ability to correctly pick out those without the disease (Lalkhen *et al.*). However, in this case, the sensitivity applies to GBM and the specificity refers to the ability to identify lymphoma. For this analysis, true positives result from a patient with GBM with five or more spectra out of the nine spectra collected correctly identified, whereas true negatives refer to the patients with lymphoma who has at least five out of the nine spectra correctly identified. False positives are where a lymphoma patient has five or more spectra incorrectly identified as GBM, and a false negative is from a patient with GBM who has five or more spectra incorrectly classified as lymphoma.

$$Sensitivity = \frac{true\ positives}{true\ positives + false\ negatives} \qquad (1)$$

$$Specificity = \frac{true\ negatives}{true\ negatives + false\ positives} \qquad (2)$$

In order to understand the reliability of the diagnostic model the Kappa value, $\kappa$, can give a quantitative measure of the magnitude of agreement between observers (Eq. 3).

$$\kappa = \frac{p_o - p_e}{1 - p_e} \qquad (3)$$

Where $p_o$ is the relative observed agreement and $p_e$ is the hypothetical probability of the chance agreement. Values of $\kappa$ range between zero and one and equate to the level of agreement. Where $\kappa$ is $\leq 0$ it indicates no agreement, 0.01-0.20 accounts for slight, 0.21-0.40 fair, moderate agreement is 0.41-0.60, 0.61-0.80 is substantial and lastly 0.8-1.00 is almost perfect agreement (Viera *et al.,* McHugh).

An n-fold cross validation was performed (n = 5) on the training data to determine the optimum values for the tuning parameters. Due to the slight class imbalance present when examining the difference between GBM (71 patients) vs. lymphoma (41 patients), various sampling methods were used throughout this study to ensure no bias was present within the models; up-sampling, down-sampling and synthetic minority over-sampling technique (SMOTE). The up-sampling method consists of repeatedly sampling the minority class to increase the number of samples, whereas down-sampling selects a subset of the majority class at random, removing the extra samples to make it the same size as the minority class (Simafore). SMOTE is unique in that it artificially mixes the data to, create 'new' samples to achieve a more balanced dataset (Chawla *et al.*).

Random forest

RF is a robust machine learning technique that builds an ensemble of decision trees from the training data using the Classification and Regression Trees (CART) algorithm (Breiman *et al.*). The RF analysis can extract statistical values, based on the number of true positives, false positives, true negatives and false negatives, determining both the accuracy and reliability of the classification. Additionally, spectral importance results can be graphically viewed in the form of Gini plots. Using the Gini impurity metric, produced from the combined mean decrease in the Gini coefficient with respect to the wavenumbers, RF can rank the spectral features in order of significance - for example, which wavenumbers are the most discriminating between the two classes (Smith *et al.* (1)).

Partial least squares-discriminant analysis

Partial Least Squares - Discriminant Analysis (PLS-DA) is supervised machine learning method that combines PLS regression (PLSR) and Linear Discriminant Analysis (LDA). This technique can extract important information from complex datasets, by reducing the dimensionality to reveal hidden patterns within the data. This technique separates classes by looking for a straight line that divides the data space into two distinct regions (Ballabio *et al.*). The data points are projected perpendicularly to the line, which is known as the discriminator (Lee *et al.*). The distances from the discriminator are referred to as the discriminant scores (Brereton *et al.*). This information is provided in the form of new variables called PLS components, where the first PLS component (PLS1) accounts for the greatest variation in the dataset, PLS2 represents the next greater variation, and so on. PLS scores plots give an overview of the general inconsistences within large datasets, and loadings plots further explain the variance, by suggesting where the most variable regions exist e.g. which spectral regions display the highest disparity.

Support vector machine

A support vector machine (SVM) is a supervised algorithm, commonly employed for classification purposes (Cortes *et al.*). From known data, SVM outputs an optimal dimension for the separation of the data, known as the hyperplane. Support vectors are the co-ordinates of the individual observation and the hyperplane can be used to categorise new samples (de Boves Harrington). The optimization of SVM tuning parameters can change the classification efficiency dramatically. The cost, C, can be referred to as the penalty parameter and is responsible for the trade-off between smooth boundaries and the ability to classify the data. The gamma parameter, $\gamma$, is responsible for the level of fit. It is important to ensure the model does not overfit the data, which is achieved using a grid search to identify the optimal classification performance (Ben-Hur *et al.*).

Centrifugal Filtration

To assess whether ATR-FTIR spectroscopy could detect *IDH1* mutation, centrifugal filtration was undertaken to enable analysis of the low molecular weight (LMW) fraction of the serum samples. The whole serum from the 72 brain cancer patients were filtered to remove the more abundant high molecular weight (HMW) biomolecules. Commercially available Amicon Ultra-0.5 mL centrifugal filtering devices (Millipore-Merck, Germany) with cut-off points at 3kDa were used to fractionate the serum samples. The serum was split into two fractions; the 'filtrate' and the 'concentrate'. The filtrate accounts for the biomolecular components below the 3kDa cut-off point, and the concentrate represents the higher MW serum constituents. Serum from each patient (0.3 mL) was placed in the centrifugal filters, and the filtration tubes were centrifuged for 30 minutes at a speed of 14000 g. The filtrates passed through the membranes into the collection vials. The filters were then inverted and centrifuged for 2 minutes at 1000 g to collect the HMW concentrates. The filtrates and concentrates were stored in a -80 °C freezer until the time of analysis.

For centrifugal filtration study, spectra were initially corrected with extended multiplicative signal correction (EMSC) using an averaged filtrate spectrum as the reference (see Kohler et al, for example). As there were two prominent bands present between 1000-800 $cm^{-1}$ in the filtered serum spectrum, the dataset was cut down to 800 $cm^{-1}$ to ensure all potentially important biological information was retained. Thus, three spectral cuts were tested; 4000-800 $cm^{-1}$, 1800-800 $cm^{-1}$ and 1800-1000 $cm^{-1}$. All other parameters were the consistent from the whole serum analysis.

## Results

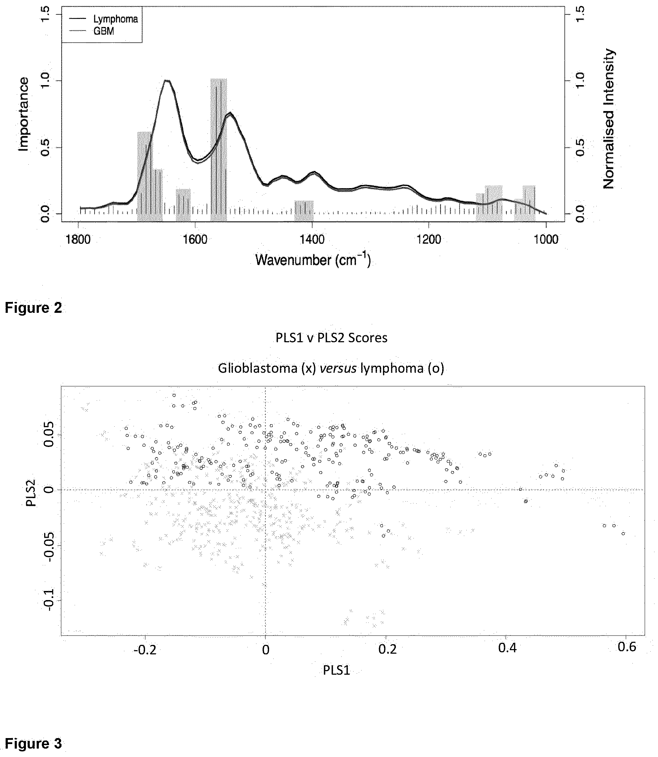

An initial random forest (RF) model provides us with the biochemical differences between the lymphoma and GBM patients. The Gini plot (Figure 2) suggests the Amide II region is of importance, closely followed by the Amide I band. Between 1150-1000$cm^{-1}$ there are various significant bands, relating to vibrations within nucleic material, glycogen and carbohydrates (Table 3).

**Table 3 - Top 15 wavenumbers from RF classification of lymphoma vs GBM with tentative biochemical assignments (Baker et *al.,* Movasaghi et *al.*)**

| Wavenumbers ($cm^{-1}$) | $\Sigma$Gini | Tentative Assignments | Vibrational Modes |
|---|---|---|---|
| 1556.5 | 95.9 | Amide II of proteins | $\delta$(N-H), v(C-N), $\delta$(C-O), v(C-C) |
| 1564.5 | 91.4 | | |
| 1676.5 | 57.9 | Amide I of proteins | v(C=O), v(C-N), $\delta$(N-H) |
| 1684.5 | 50.1 | | |
| 1572.5 | 42.9 | Amide II of proteins | $\delta$(N-H), v(C-N), $\delta$(C-O), v(C-C) |
| 1548.5 | 32.6 | | |
| 1668.5 | 32.2 | Amide I of proteins | v(C=O), v(C-N), $\delta$(N-H) |
| 1660.5 | 30.5 | | |
| 1020.5 | 19.7 | DNA/Glycogen | v($PO_2^-$)/v(C-O), def(C-OH) |
| 1100.5 | 19.0 | Nucleic Acids | v($PO_2^-$) |
| 1036.5 | 17.4 | Glycogen | v(C-O), v(C-C) |
| 1692.5 | 15.3 | Amide I of proteins | v(C=O), v(C-N), $\delta$(N-H) |
| 1108.5 | 14.6 | Carbohydrate | v(C-O), v(C-C) |
| 1628.5 | 14.5 | Amide I of proteins | v(C=O), v(C-N), $\delta$(N-H) |
| 1620.5 | 13.2 | | |
| v = stretching; $\delta$ = bending; def = deformation | | | |

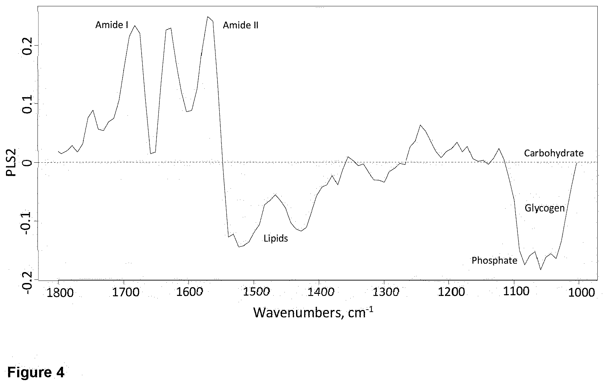

It was found that the PLS-DA scores plot separates the lymphoma and GBM patients across the 2nd PLS competent (Figure 3). Again, we see the highest discrimination arises from the Amide II band and the lower wavenumber region on the loadings plot (Figure 4). For lymphoma vs GBM, the Amide I region is also highly discriminatory, substantiating the RF Gini findings outlined previously in Table 3.

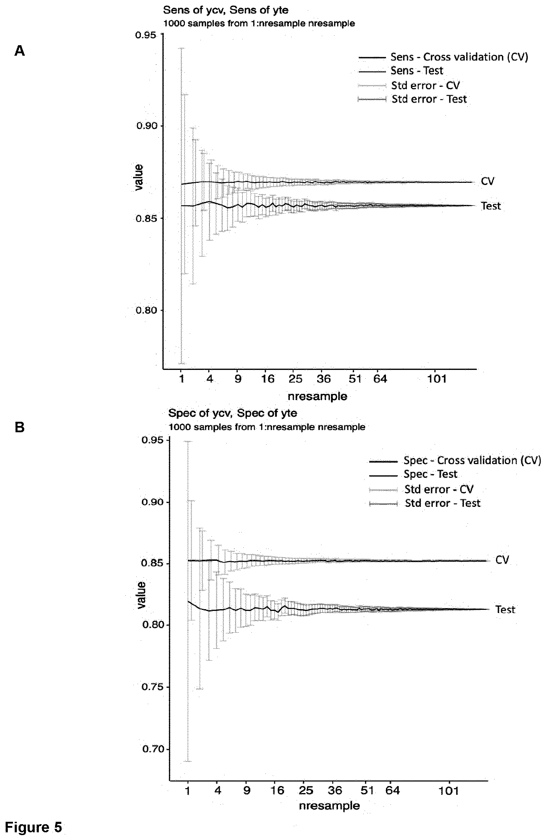

Bootstrapping analysis was done on the lymphoma vs GBM training set to search for an acceptable number of iterations. In this case, 51 resamples were also found to be sufficient, with the standard error converging at this point (Figure 5).

SMOTE showed to be the best sampling technique for RF and PLS-DA, but up-sampling was found to be optimal for the SVM-based model (Table 4).

**Table 4 - Statistical results for the lymphoma vs GBM test sets from the three different classification models with 51 iterations**

| | RF + SMOTE | | | PLS-DA + SMOTE | | | SVM + UP | | |
|---|---|---|---|---|---|---|---|---|---|
| | Mean | Optimum | SD | Mean | Optimum | SD | Mean | Optimum | SD |
| **Kappa** | 0.63 | 0.94 | 0.13 | 0.76 | 0.94 | 0.09 | 0.72 | 0.94 | 0.11 |
| **Sensitivity (%)** | 90.9 | 100 | 5.8 | 90.1 | 100 | 5.7 | 86.6 | 100 | 8.5 |
| **Specificity (%)** | 70.8 | 100 | 14.9 | 86.3 | 100 | 9.4 | 86.3 | 100 | 9.5 |
| **Accuracy (%)** | 80.8 | 97.6 | 7.2 | 88.2 | 97.6 | 5.0 | 86.4 | 97.6 | 5.4 |

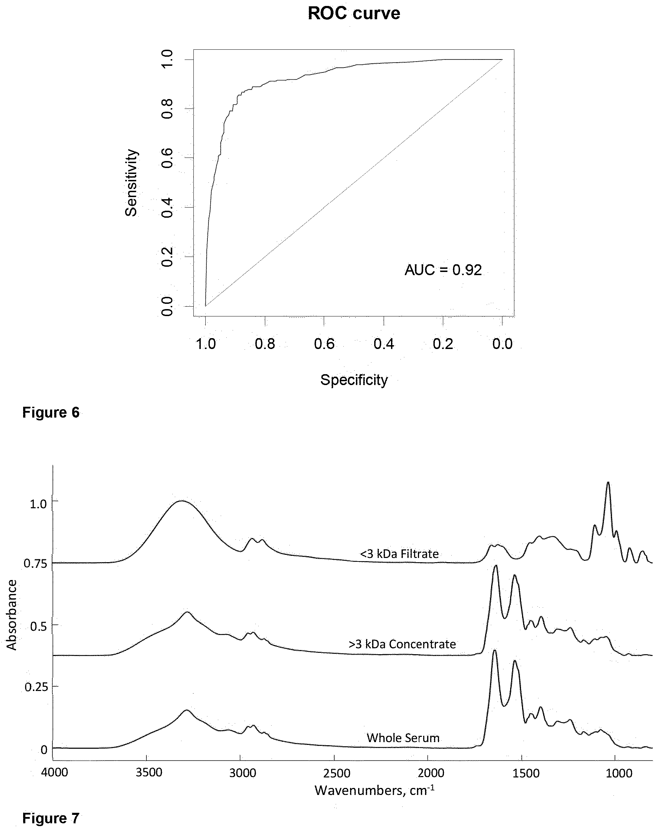

For this particular dataset, the sensitivities refer to the ability to detect GBM, and the specificity relates to lymphoma. As shown in Table 4 the least effective model for this dataset was found to be RF - despite having a high sensitivity, the specificity was rather low at 70.8%. SVM combined with up-sampling performed well, reporting a balanced accuracy of 86.4%. The PLS-DA + SMOTE method seemed to be the optimal model, with a sensitivity of 90.1%, a specificity of 86.3%, and the highest $\kappa$ value of all three models - mean $\kappa$ = 0.76. Each technique reported 100% for sensitivity and specificity for at least one of the 51 iterations. The sensitivities were relatively stable, but the predictions for lymphoma were more variable, for example, one of the RF resamples reported a sensitivity of 42%, which ultimately lowered the mean value. That said, the ROC curve for the SVM-based model still indicates promising diagnostic capability, with an AUC value of 0.92 (Figure 6).

**ATR- FITR IDH analysis**

Brain cancer patients - with either astrocytoma, oligodendroglioma or GBM - were separated based upon their *IDH1* status using ATR-FTIR serum spectroscopy. Of the 72 patients included, there were 36 with the *IDH1* mutation, and 36 *IDH1*-wildtype. The data was classified through RF, PLS-DA and SVM with 100 resamples for each, and the findings are reported in Table 6 on a 'by patient' basis. For the whole serum dataset, the SVM model reported a promising sensitivity of 75.9% but had an extremely low specificity of 28%. All models seemed to be more effective at picking out the *IDH1*-mutated serum samples from the test sets, as the sensitivities were much higher than the specificities in each case. It is not clear why this may be, as there were an equal number of samples in each class and therefore should be no bias present in the models. That being said, the results did not appear to be reliable, and given the poor balanced accuracies (-50%) it could be assumed the correct predictions were ultimately made by chance.

Table 6 - Classification results for the *IDH1*-mutated versus *IDH1*-wildtype whole serum dataset, after 100 resamples. The mean sensitivity, specificity and balanced accuracy are reported with their corresponding standard deviations (SD).

| Sample fraction | Model | Sensitivity (%) | | Specificity (%) | | Balanced accuracy (%) | |
|---|---|---|---|---|---|---|---|
| | | Mean | SD | Mean | SD | Mean | SD |
| *Whole Serum* | RF | 50.3 | 15.2 | 45.4 | 15.1 | 47.9 | 8.6 |
| | PLS-DA | 69.3 | 13.8 | 35.3 | 14.7 | 52.3 | 7.4 |
| | SVM | 75.9 | 17.5 | 28.0 | 14.6 | 51.9 | 7.7 |

Blood serum constitutes thousands of different proteins, ranging from the more abundant HMW serum albumin (50 g/L) to the LMW proteins like troponin (1 ng/L). Due to the wealth of various biomolecules that exist in a normal serum sample, it was expected to be a significant challenge to identify the subtle alterations in blood composition, that may have been associated with the *IDH1* mutation. The LMW fraction of serum is believed to contain disease-specific information, making the spectroscopic signature of this fraction useful for diagnostics. Thus, after the poor classification performance for the whole serum data, it was thought that discrete molecular differences could potentially be emphasised through the use of centrifugal filtration.

Figure 7 provides an example of the IR spectra for whole serum, the >3kDa 'HMW' fraction and the <3kDa 'LMW' fraction. The concentrate appears almost identical to the whole serum spectrum; notably, they have a very similar absorbance from the more abundant proteins - such as albumin and immunoglobulins - that exist within the Amide region. With these large proteins and other HMW constituents removed, the filtrate spectrum looks remarkably different, with only a few distinct peaks in the fingerprint region (red spectrum). Three spectral regions were chosen for examination: 4000-800 cm$^{-1}$ and 1800-800 cm$^{-1}$ - to encompass the two distinct peaks around 950 cm$^{-1}$ and 850 cm$^{-1}$ - as well as the typical biological fingerprint region (1800-1000 cm$^{-1}$). The classification results are reported in Table 7.

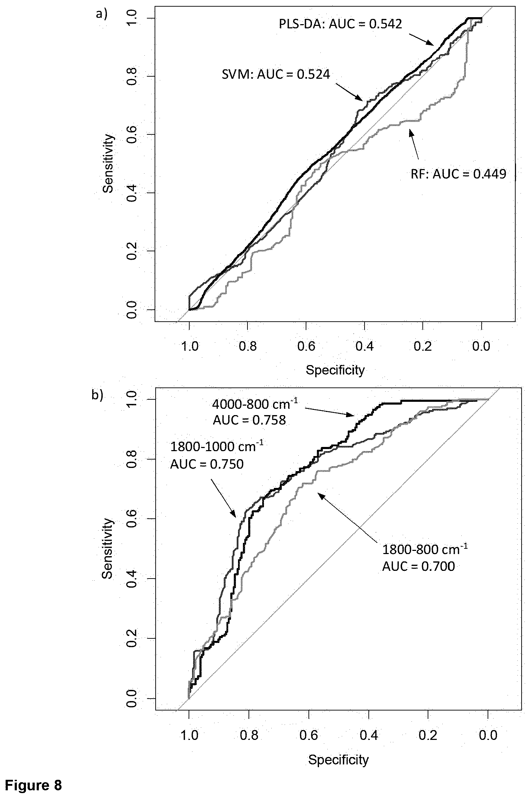

In each case, the filtrate models were superior to the whole serum models in successfully detecting the *IDH1*-wildtype patients, reporting specificity values above 60%. The improvement in diagnostic ability due to the filtration step is emphasised in Figure 8, which displays single model ROC curves for the three whole serum classifiers (Figure 8a) and the best models for each of the three filtrate datasets (Figure 8b). As expected from the poor classification results, the ROC curves for the whole serum models fall on the diagonal line, meaning the predictions that are being made are no better than random guessing, and the reported AUC values of ~0.5 suggests the test has essentially no diagnostic accuracy. However, the inclusion of centrifugal filtration enhanced the ability to successfully discriminate the two *IDH1* classes. The corresponding ROC curves in Figure 8b report AUC values >0.7, which is often deemed an 'acceptable' level of discrimination.

Table 7 - Classification results for the *IDH1*-mutated versus *IDH1*-wildtype serum datasets after 100 resamples. The mean sensitivity, specificity and balanced accuracy are reported with their corresponding standard deviations (SD). Best performing models for every sample fraction is highlighted in bold.

| Sample fraction | Model | Sensitivity (%) | | Specificity (%) | | Balanced accuracy (%) | |
|---|---|---|---|---|---|---|---|
| | | Mean | SD | Mean | SD | Mean | SD |
| <3kDa Filtered Serum (4000-800 cm$^{-1}$) | RF | 68.4 | 16.2 | 67.5 | 15.9 | 68.0 | 11.1 |
| | **PLS-DA** | **75.5** | 12.3 | **62.6** | 15.5 | **69.1** | 9.0 |
| | SVM | 68.4 | 16.5 | 64.2 | 16.0 | 66.4 | 10.2 |
| <3kDa Filtered Serum (1800-800 cm$^{-1}$) | **RF** | **70.6** | 17.8 | 66.4 | 14.5 | **68.5** | 11.2 |
| | PLS-DA | 65.0 | 14.6 | 64.6 | 16.5 | 64.8 | 8.7 |
| | SVM | 63.2 | 16.3 | 63.8 | 16.9 | 63.5 | 9.6 |
| <3kDa Filtered Serum (1800-1000 cm$^{-1}$) | **RF** | **66.6** | 15.4 | **68.1** | 14.1 | **67.4** | 9.9 |
| | PLS-DA | 65.9 | 14.6 | 56.2 | 15.5 | 61.1 | 9.1 |
| | SVM | 68.1 | 15.6 | 56.8 | 15.6 | 62.5 | 10.1 |

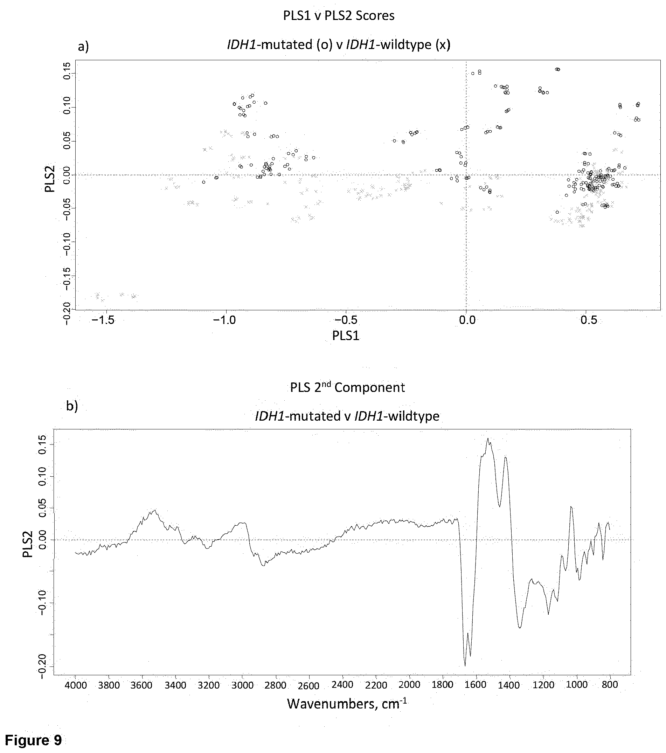

The <3kDa filtered serum 'full spectra' dataset (4000-800 cm$^{-1}$) delivered the greatest balanced accuracy of 69.1% when classified by the PLS-DA model. The PLS scores plot in Figure 9a describes the general variation within the dataset. The major variance is generally described by the first PLS component (PLS1). The PLS1 loadings suggest large differences -3400 cm$^{-1}$ and ~1650 cm$^{-1}$, although there is no apparent class separation across PLS1 in the scores plot. Despite some overlap, it is evident that the 2nd PLS component separates the two classes better than PLS1. The PLS2 loadings also highlight significant spectral differences around -1650 cm$^{-1}$ (Figure 9b). Interestingly, this is the typical location of the large Amide I band in a normal serum spectrum, accounting for the bond vibrations within an abundance of protein molecules. Even with the HMW proteins filtered out of the samples - like albumin and immunoglobulins - it still appears to be a region of importance when examining molecules of very low molecular weights (<3kDa), suggesting the smaller protein molecules still have diagnostic potential.

In general, the balanced accuracies were enhanced to between 60-70% for all tested models. The centrifugal filtration step has produced a significant improvement on the model performance, by delivering more balanced sensitivities and specificities.

## Conclusion

The implementation of a quick blood serum test for the early detection of brain tumours at a GP setting could have a huge impact on the quality of life and prognosis for patients.

We present the ability of the method of the invention to differentiate between brain tumour types. Notably, the separation of lymphoma and GBM through ATR-FTIR spectroscopy would be particularly attractive for neurologists in a secondary care setting, when imaging results are not clear. This proof-of-principle study involved 112 patients, providing a sensitivity of 90.1% and a specificity of 86.3%. A $\kappa$ value of 0.76 indicates the technique is reliable.

Identification of the molecular status from blood serum prior to biopsy could further direct some patients to alternative treatment strategies. Initially, the whole serum classifiers performed poorly, delivering balanced accuracies of -50%. Yet with the introduction of centrifugal filtration the classification performance improved significantly, enhancing the sensitivities and specificities to around 70%. These strategies may be further optimised in prospective clinical studies, and can be extended to identify other important molecular alterations, such as *ATRX* loss, *1p/19q* co-deletion and/or *MGMT* hypermethylation, with which brain cancer type can be stratified pre-operatively.

**REFERENCES**

M. J. Baker et al., 'Using Fourier transform IR spectroscopy to analyse biological materials', Nature Protocols, vol. 9, no. 8, pp. 1771-1791, Jul. 2014.

D. Ballabio and V. Consonni, 'Classification tools in chemistry. Part 1: linear models. PLS-DA', Analytical Methods, vol. 5, no. 16, p. 3790, 2013.

A. Barth, Biochimica et Biophysica Acta 1767 (2007) 1073-1101

A. Ben-Hur and J. Weston, 'A User's Guide to Support Vector Machines', in Data Mining Techniques for the Life Sciences, vol. 609, O. Carugo and F. Eisenhaber, Eds. Totowa, NJ: Humana Press, 2010, pp. 223-239.

L. Breiman, 'Random Forests', Machine Learning, vol. 45, no. 1, pp. 5-32, Oct. 2001. R. G. Brereton and G. R. Lloyd, 'Partial least squares discriminant analysis: taking the magic away', Journal of Chemometrics, vol. 28, no. 4, pp. 213-225, Apr. 2014.

N. V. Chawla, K. W. Bowyer, L. O. Hall, and W. P. Kegelmeyer, 'SMOTE: Synthetic Minority Over-sampling Technique', Journal of Artificial Intelligence Research, vol. 16, pp. 321-357, Jun. 2002.

C. Cortes and V. Vapnik, 'Support-Vector Networks', Machine Learning, vol. 20, no. 3, pp. 273-297, Sep. 1995.

S.E. Glassford et al. Biochimica et Biophysica Acta 1834 (2013) 2849-2858P. de Boves Harrington, 'Support Vector Machine Classification Trees', Anal. Chem., vol. 87, no. 21, pp. 11065-11071, Nov. 2015.

A. Kohler, C. Kirschner, A. Oust, H. Martens, Extended multiplicative signal correction as a tool for separation and characterization of physical and chemical information in Fourier transform infrared microscopy images of cryo-sections of beef loin, Applied Spectroscopy 59 (2005) 707-716

A. G. Lalkhen and A. McCluskey, 'Clinical tests: sensitivity and specificity', Continuing Education in Anaesthesia Critical Care & Pain, vol. 8, no. 6, pp. 221-223, Dec. 2008.

L. C. Lee, C.-Y. Liong, and A. A. Jemain, 'Partial least squares-discriminant analysis (PLS-DA) for classification of high-dimensional (HD) data: a review of contemporary practice strategies and knowledge gaps', Analyst, vol. 143, no. 15, pp. 3526-3539, 2018.

M. L. McHugh, 'Interrater reliability: the kappa statistic', Biochem Med (Zagreb), vol. 22, no. 3, pp. 276-282, 2012.

Z. Movasaghi, S. Rehman, and Dr. I. ur Rehman, 'Fourier Transform Infrared (FTIR) Spectroscopy of Biological Tissues', Applied Spectroscopy Reviews, vol. 43, no. 2, pp. 134-179, Feb. 2008.

SIMAFORE, 'Managing unbalanced data for building machine learning models', Mar-2019. [Online]. Available: http://www.simafore.com/blog/handling-unbalanced-data-machine-learning-models.

(1) B. R. Smith et al., 'Combining random forest and 2D correlation analysis to identify serum spectral signatures for neuro-oncology', Analyst, vol. 141, no. 12, pp. 3668-3678, 2016.

(2) B. R. Smith, M. J. Baker, and D. S. Palmer, 'PRFFECT: A versatile tool for spectroscopists', Chemometrics and Intelligent Laboratory Systems, vol. 172, pp. 33-42, Jan. 2018.

A. J. Viera and J. M. Garrett, 'Understanding Interobserver Agreement: The Kappa Statistic', Family Medicine, p. 4.

**Claims**

1. A method of classifying whether a subject suspected of having a brain tumour has a glioma, such as glioblastoma multiforme, or a central nervous system lymphoma, such as primary cerebral lymphoma, the method comprising:

    performing spectroscopic analysis upon a blood sample, or component thereof, such as plasma or serum, isolated from the subject to obtain a spectroscopic signature characteristic of the blood sample (or component thereof); wherein the spectroscopic analysis is Attenuated Total Reflection FTIR (ATR-FTIR); and classifying the subject as having glioma or a central nervous system lymphoma using the obtained spectroscopic signature characteristic of the blood sample (or component thereof).

2. The method according to claim 1, wherein the spectroscopic signature characteristic of the blood sample (or component thereof) is the spectrum between 400 and 4000 $cm^{-1}$, 900 and 1800 $cm^{-1}$, or 1400 and 1800 $cm^{-1}$..

3. The method according to claims 1 or 2, wherein a silicon internal reflection element supports the blood sample, or component thereof, during spectroscopic analysis.

4. The method according to claims 1 or 2, wherein "ATR crystals" or a silicon internal reflection element (IRE) support the blood sample, or component thereof, during spectroscopic analysis.

5. The method according to any one of the preceding claims, wherein the subject is classified as having glioma when at least a portion of the obtained spectroscopic signature characteristic of the blood sample, or component thereof, is lower than at least a portion of a control spectroscopic signature, and the subject is classified as having central nervous system lymphoma when at least a portion of the obtained spectroscopic signature characteristic of the blood sample, or component thereof, is higher than at least a portion of a control spectroscopic signature, optionally wherein the control spectroscopic signature comprises a plurality of pre-correlated signatures stored in a database, e.g. a "training set", in order to derive a correlation with a determination of glioma or central nervous system lymphoma.

6. The method according to any one of claims 1 to 5, wherein classifying whether the subject has a glioma or a central nervous system lymphoma using the obtained spectroscopic signature characteristic of the blood sample, or component thereof, comprises correlating the obtained spectroscopic signature with a determination of glioma or central nervous system lymphoma based on a predictive model developed by "training", e.g. via pattern recognition algorithms, a database of pre-correlated analyses.

7. The method according to any one of the preceding claims, wherein classifying the subject as having glioma or central nervous system lymphoma using the obtained spectroscopic signature characteristic of the blood sample, or component thereof, facilitates a determination of treatment of the subject.

8. The method according to any of claims 6 - 7, wherein if the subject is classified as having glioma this facilitates the determination of at least one treatment of the subject and if the subject is classified as having central nervous system lymphoma this facilitates the determination of at least one other different treatment of the subject, optionally wherein treatment with chemotherapy and/or radiotherapy is selected if the subject is determined to have central nervous system lymphoma and treatment with surgery is selected if the subject is determined to have the glioma and optionally where biological marker status aids a surgeon in determining a degree of resection to carry out.

9. A diagnostic kit for classifying whether a subject suspected of having a brain tumour has a glioma or a central nervous system lymphoma, the kit comprising a device configured to receive a blood sample, or component thereof, from the subject and to perform spectroscopic analysis upon the blood sample, or component thereof, of the subject to produce a spectroscopic signature characteristic of the blood sample (or component thereof); and a device to classify the subject as having a glioma or a central nervous system lymphoma using the obtained spectroscopic signature characteristic of the blood sample (or component thereof); wherein the spectroscopic analysis is Attenuated Total Reflection FTIR (ATR-FTIR), wherein the device to classify the subject as having a glioma or a central nervous system lymphoma comprises or is in communication with a computer, which computer is installed with computer software configured to operate the computer to perform a classification in relation to glioma and central nervous system lymphoma based on an ATR-FTIR spectroscopic signature of a blood sample of a subject.

10. The diagnostic kit of claim 9, wherein the device configured to receive a blood sample is configured to automatically prepare a blood sample (or component thereof) of a required thickness and dryness, and/or wherein the device for performing spectroscopic analysis upon the blood sample (or component thereof) is the same as the device to classify the subject as having a glioma or a central nervous system lymphoma.

11. A computer-readable medium comprising the computer software mentioned in claim 9.

**Patentansprüche**

1. Verfahren zur Klassifizierung, ob ein Proband, bei welchem der Verdacht auf einen Hirntumor besteht, ein Gliom, wie z. B. ein Glioblastoma multiforme, oder ein Lymphom des Zentralnervensystems, wie z. B. ein primäres zerebrales Lymphom, hat, wobei das Verfahren Folgendes umfasst:

    Durchführen einer spektroskopischen Analyse einer Blutprobe oder einer Komponente davon, wie z. B. Plasma oder Serum, die von dem Probanden isoliert wurde, um eine spektroskopische Signatur zu erzielen, die für die Blutprobe (oder eine Komponente davon) charakteristisch ist; wobei die spektroskopische Analyse eine abgeschwächte Totalreflexions-FTIR (ATR-FTIR) ist; und
    Klassifizieren des Probanden als Proband mit einem Gliom oder einem Lymphom des Zentralnervensystems unter Verwendung der erzielten spektroskopischen Signatur, die für die Blutprobe (oder eine Komponente davon) charakteristisch ist.

2. Verfahren nach Anspruch 1, wobei die für die Blutprobe (oder eine Komponente davon) charakteristische spektroskopische Signatur das Spektrum zwischen 400 und 4 000 cm$^{-1}$, 900 und 1 800 cm$^{-1}$ oder 1 400 und 1 800 cm$^{-1}$ ist.

3. Verfahren nach Anspruch 1 oder 2, wobei ein Silizium-Innenreflexionselement die Blutprobe oder die Komponente davon während der spektroskopischen Analyse trägt.

4. Verfahren nach Anspruch 1 oder 2, wobei während der spektroskopischen Analyse die Blutprobe oder die Komponente davon durch "ATR-Kristalle" oder ein internes Siliziumreflexionselement (IRE) getragen wird.

5. Verfahren nach einem der vorhergehenden Ansprüche, wobei der Proband als Proband mit einem Gliom klassifiziert wird, wenn mindestens ein Abschnitt der erzielten spektroskopischen Signatur, die für die Blutprobe oder die Komponente davon charakteristisch ist, niedriger ist als mindestens ein Abschnitt einer spektroskopischen Kontrollsignatur, und der Proband als Proband mit einem Lymphom des Zentralnervensystems klassifiziert wird, wenn mindestens ein Abschnitt der erzielten spektroskopischen Signatur, die für die Blutprobe oder die Komponente davon charakteristisch ist, höher ist als mindestens ein Abschnitt einer spektroskopischen Kontrollsignatur, wobei die spektroskopische Kontrollsignatur optional eine Vielzahl von vorkorrelierten Signaturen umfasst, die in einer Datenbank, z. B. in einem "Trainingssatz", gespeichert sind, um eine Korrelation mit einer Bestimmung eines Glioms oder eines Lymphoms des Zentralnervensystems abzuleiten.

6. Verfahren nach einem der Ansprüche 1 bis 5, wobei die Klassifizierung, ob der Proband ein Gliom oder ein Lymphom des Zentralnervensystems hat, unter Verwendung der erzielten spektroskopischen Signatur, die für die Blutprobe oder die Komponente davon charakteristisch ist, das Korrelieren der erzielten spektroskopischen Signatur mit einer Bestimmung eines Glioms oder eines Lymphoms des Zentralnervensystems auf der Grundlage eines Vorhersagemodells umfasst, das durch "Trainieren", z. B. über Algorithmen zur Mustererkennung, einer Datenbank mit vorkorrelierten Analysen entwickelt wurde.

7. Verfahren nach einem der vorhergehenden Ansprüche, wobei die Klassifizierung des Probanden als Proband mit einem Gliom oder einem Lymphom des Zentralnervensystems unter Verwendung der erzielten spektroskopischen Signatur, die für die Blutprobe oder die Komponente davon charakteristisch ist, eine Bestimmung der Behandlung des Probanden erleichtert.

8. Verfahren nach einem der Ansprüche 6 bis 7, wobei, wenn der Proband als Proband mit Gliom klassifiziert wird, dies die Bestimmung mindestens einer Behandlung des Probanden erleichtert, und wenn der Proband als Proband mit einem Lymphom des Zentralnervensystems klassifiziert wird, dies die Bestimmung mindestens einer anderen unterschiedlichen Behandlung des Probanden erleichtert, wobei optional eine Behandlung mit Chemotherapie und/oder Strahlentherapie ausgewählt wird, wenn der Proband als Proband mit einem Lymphom des Zentralnervensystems bestimmt wird, und eine Behandlung mit einem chirurgischen Eingriff ausgewählt wird, wenn der Proband als Proband mit einem Gliom bestimmt wird, und worin optional ein biologischer Markerstatus einem Chirurgen bei der Bestimmung eines durchzuführenden Resektionsgrades hilft.

9. Diagnostik-Kit zur Klassifizierung, ob ein Proband, bei welchem der Verdacht auf einen Hirntumor besteht, ein Gliom oder ein Lymphom des Zentralnervensystems hat, wobei der Kit eine Vorrichtung umfasst, die so konfiguriert ist, dass sie eine Blutprobe oder eine Komponente davon von der Person aufnimmt und eine spektroskopische Analyse der Blutprobe oder der Komponente davon des Probanden durchführt, um eine für die Blutprobe (oder die Komponente davon) charakteristische spektroskopische Signatur zu erzeugen; und eine Vorrichtung zum Klassifizieren des Probanden als Proband mit einem Gliom oder einem Lymphom des Zentralnervensystems unter Verwendung der erzielten spektroskopischen Signatur, die für die Blutprobe (oder die Komponente davon) charakteristisch ist; wobei die spektroskopische Analyse eine abgeschwächte Totalreflexions-FTIR (ATR-FTIR) ist, wobei die Vorrichtung zur Klassifizierung des Probanden als Proband mit einem Gliom oder einem Lymphom des Zentralnervensystems einen Computer umfasst oder mit diesem in Verbindung steht, wobei der Computer mit einer Computersoftware installiert ist, die so konfiguriert ist, dass sie den Computer betreibt, um eine Klassifizierung in Bezug auf Gliom und Lymphom des Zentralnervensystems auf der Grundlage einer ATR-FTIRspektroskopischen Signatur einer Blutprobe eines Probanden durchzuführen.

10. Diagnostik-Kit nach Anspruch 9, wobei die Vorrichtung, die zur Aufnahme einer Blutprobe konfiguriert ist, so konfiguriert ist, dass sie automatisch eine Blutprobe (oder eine Komponente davon) mit einer erforderlichen Dicke und Trockenheit präpariert, und/oder wobei die Vorrichtung zur Durchführung einer spektroskopischen Analyse der Blutprobe (oder der Komponente davon) dieselbe ist wie die Vorrichtung zur Klassifizierung des Probanden als Proband mit einem Gliom oder einem Lymphom des Zentralnervensystems.

11. Computerlesbares Medium, umfassend die in Anspruch 9 genannte Computersoftware.

## Revendications

1. Procédé permettant de classifier si un sujet soupçonné d'avoir une tumeur cérébrale a un gliome, tel qu'un glioblastome multiforme, ou un lymphome du système nerveux central, tel qu'un lymphome cérébral primitif, le procédé comprenant les étapes consistant à

   effectuer une analyse spectroscopique sur un échantillon de sang, ou un composant de celui-ci, tel que du plasma ou du sérum, isolé du sujet pour obtenir une signature spectroscopique caractéristique de l'échantillon de sang (ou du composant de celui-ci) ; l'analyse spectroscopique étant une FTIR à réflexion totale atténuée (ATR-FTIR) ; et
   classifier le sujet comme un sujet ayant un gliome ou un lymphome du système nerveux central à l'aide de la signature spectroscopique obtenue, caractéristique de l'échantillon de sang (ou du composant de celui-ci).

2. Procédé selon la revendication 1, dans lequel la signature spectroscopique caractéristique de l'échantillon de sang (ou du composant de celui-ci) est le spectre compris entre 400 et 4000 $cm^{-1}$, 900 et 1800 $cm^{-1}$ ou 1400 et 1800 $cm^{-1}$.

3. Procédé selon la revendication 1 ou 2, dans lequel un élément de réflexion interne en silicium supporte l'échantillon de sang, ou le composant de celui-ci, lors de l'analyse spectroscopique.

4. Procédé selon la revendication 1 ou 2, dans lequel des « cristaux ATR » ou un élément de réflexion interne en silicium (IRE) supportent l'échantillon de sang, ou le composant de celui-ci, lors de l'analyse spectroscopique.

5. Procédé selon l'une quelconque des revendications précédentes, dans lequel le sujet est classifié comme sujet ayant un gliome lorsqu'au moins une partie de la signature spectroscopique obtenue, caractéristique de l'échantillon de sang ou du composant de celui-ci, est inférieure à au moins une partie d'une signature spectroscopique témoin, et le sujet est classifié comme sujet ayant un lymphome du système nerveux central lorsqu'au moins une partie de la signature spectroscopique obtenue, caractéristique de l'échantillon de sang ou du composant de celui-ci, est supérieure à au moins une partie d'une signature spectroscopique témoin, dans lequel, facultativement, la signature spectroscopique témoin comprend une pluralité de signatures pré-corrélées stockées dans une base de données, par ex. un « ensemble d'apprentissage », afin de dériver une corrélation avec une détermination d'un gliome ou d'un lymphome du système nerveux central.

6. Procédé selon l'une quelconque des revendications 1 à 5, dans lequel l'étape consistant à classifier si le sujet a un gliome ou un lymphome du système nerveux central à l'aide de la signature spectroscopique obtenue, caractéristique de l'échantillon de sang ou du composant de celui-ci, comprend la corrélation de la signature spectroscopique obtenue avec une détermination de gliome ou de lymphome du système nerveux central reposant sur un modèle prédictif développé par « apprentissage », par ex. par des algorithmes de reconnaissance des motifs, d'une base de données d'analyses pré-corrélées.

**7.** Procédé selon l'une quelconque des revendications précédentes, dans lequel la classification du sujet comme ayant un gliome ou un lymphome du système nerveux central à l'aide de la signature spectroscopique obtenue, caractéristique de l'échantillon de sang ou du composant de celui-ci, facilite une détermination du traitement du sujet.

**8.** Procédé selon l'une quelconque des revendications 6 et 7, dans lequel si le sujet est classifié comme ayant un gliome, cela facilite la détermination d'au moins un traitement du sujet et si le sujet est classifié comme ayant un lymphome du système nerveux central, cela facilite la détermination d'au moins un autre traitement différent du sujet, dans lequel, facultativement, un traitement par chimiothérapie et/ou radiothérapie est choisi s'il est déterminé que le sujet a un lymphome du système nerveux central et un traitement chirurgical est choisi s'il est déterminé que le sujet a un gliome et facultativement où un statut de marqueur biologique aide le chirurgien à déterminer un degré de résection à effectuer.

**9.** Nécessaire de diagnostic pour classifier si un sujet soupçonné d'avoir une tumeur cérébrale a un gliome ou un lymphome du système nerveux central, le nécessaire comprenant un dispositif configuré pour recevoir un échantillon de sang, ou un composant de celui-ci, du sujet et pour effectuer une analyse spectroscopique sur l'échantillon de sang, ou le composant de celui-ci, du sujet pour produire une signature spectroscopique caractéristique de l'échantillon de sang (ou du composant de celui-ci) ; et un dispositif permettant de classifier le sujet comme sujet ayant un gliome ou un lymphome du système nerveux central à l'aide de la signature spectroscopique obtenue, caractéristique de l'échantillon de sang (ou du composant de celui-ci) ; ladite analyse spectroscopique étant une FTIR à réflexion totale atténuée (ATR-FTIR),
ledit dispositif permettant de classifier le sujet comme sujet ayant un gliome ou un lymphome du système nerveux central comprenant ou étant en communication avec un ordinateur, un logiciel informatique étant installé sur ledit ordinateur et étant configuré pour faire fonctionner l'ordinateur de façon qu'il effectue une classification en lien avec un gliome et un lymphome du système nerveux central en fonction d'une signature spectroscopique ATR-FTIR d'un échantillon de sang d'un sujet.

**10.** Nécessaire de diagnostic selon la revendication 9, dans lequel le dispositif configuré pour recevoir un échantillon de sang est configuré pour préparer automatiquement un échantillon de sang (ou un composant de celui-ci) d'une épaisseur et d'une siccité requises, et/ou dans lequel le dispositif permettant d'effectuer une analyse spectroscopique sur l'échantillon de sang (ou le composant de celui-ci) est le même que le dispositif permettant de classifier le sujet comme sujet ayant un gliome ou un lymphome du système nerveux central.

**11.** Support lisible par ordinateur comprenant le logiciel informatique mentionné dans la revendication 9.

A

B

**Figure 1**

**Figure 2**

PLS1 v PLS2 Scores

Glioblastoma (x) *versus* lymphoma (o)

**Figure 3**

Figure 4

**A**

Sens of ycv, Sens of yte
1000 samples from 1:nresample nresample

**B**

Spec of ycv, Spec of yte
1000 samples from 1:nresample nresample

**Figure 5**

## ROC curve

**Figure 6**

**Figure 7**

Figure 8

PLS1 v PLS2 Scores

a)

IDH1-mutated (o) v IDH1-wildtype (x)

PLS 2nd Component

b)

IDH1-mutated v IDH1-wildtype

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017221027 A1, Baker

**Non-patent literature cited in the description**

- **NAKASAWA et al.** Machine learning based on multi-parametric magnetic resonance imaging to differentiate glioblastoma multiforme from primary cerebral nervous system lymphoma. *European Journal of Radiology,* 2018, vol. 108, 1-288
- **M. J. BAKER et al.** Using Fourier transform IR spectroscopy to analyse biological materials. *Nature Protocols,* July 2014, vol. 9 (8), 1771-1791
- **D. BALLABIO ; V. CONSONNI.** Classification tools in chemistry. Part 1: linear models. PLS-DA. *Analytical Methods,* 2013, vol. 5 (16), 3790
- **A. BARTH.** *Biochimica et Biophysica Acta,* 2007, vol. 1767, 1073-1101
- A User's Guide to Support Vector Machines. **A. BEN-HUR ; J. WESTON.** Data Mining Techniques for the Life Sciences. Humana Press, 2010, vol. 609, 223-239
- **L. BREIMAN.** Random Forests. *Machine Learning,* October 2001, vol. 45 (1), 5-32
- **R. G. BRERETON ; G. R. LLOYD.** Partial least squares discriminant analysis: taking the magic away. *Journal of Chemometrics,* April 2014, vol. 28 (4), 213-225
- **N. V. CHAWLA ; K. W. BOWYER ; L. O. HALL ; W. P. KEGELMEYER.** SMOTE: Synthetic Minority Over-sampling Technique. *Journal of Artificial Intelligence Research,* June 2002, vol. 16, 321-357
- **C. CORTES ; V. VAPNIK.** Support-Vector Networks. *Machine Learning,* September 1995, vol. 20 (3), 273-297
- **S.E. GLASSFORD et al.** *Biochimica et Biophysica Acta,* 2013, vol. 1834, 2849-2858
- **P. DE BOVES HARRINGTON.** Support Vector Machine Classification Trees. *Anal. Chem.,* November 2015, vol. 87 (21), 11065-11071
- **A. KOHLER ; C. KIRSCHNER ; A. OUST ; H. MARTENS.** Extended multiplicative signal correction as a tool for separation and characterization of physical and chemical information in Fourier transform infrared microscopy images of cryo-sections of beef loin. *Applied Spectroscopy,* 2005, vol. 59, 707-716
- **A. G. LALKHEN ; A. MCCLUSKEY.** Clinical tests: sensitivity and specificity. *Continuing Education in Anaesthesia Critical Care & Pain,* December 2008, vol. 8 (6), 221-223
- **L. C. LEE ; C.-Y. LIONG ; A. A. JEMAIN.** Partial least squares-discriminant analysis (PLS-DA) for classification of high-dimensional (HD) data: a review of contemporary practice strategies and knowledge gaps. *Analyst,* 2018, vol. 143 (15), 3526-3539
- **M. L. MCHUGH.** Interrater reliability: the kappa statistic. *Biochem Med (Zagreb),* 2012, vol. 22 (3), 276-282
- **Z. MOVASAGHI ; S. REHMAN ; DR. I. UR REHMAN.** Fourier Transform Infrared (FTIR) Spectroscopy of Biological Tissues. *Applied Spectroscopy Reviews,* February 2008, vol. 43 (2), 134-179
- **SIMAFORE.** *Managing unbalanced data for building machine learning models,* March 2019, http://www.simafore.com/blog/handling-unbalanced-data-machine-learning-models
- **B. R. SMITH et al.** Combining random forest and 2D correlation analysis to identify serum spectral signatures for neuro-oncology. *Analyst,* 2016, vol. 141 (12), 3668-3678
- **B. R. SMITH ; M. J. BAKER ; D. S. PALMER.** PRFFECT: A versatile tool for spectroscopists. *Chemometrics and Intelligent Laboratory Systems,* January 2018, vol. 172, 33-42
- **A. J. VIERA ; J. M. GARRETT.** Understanding Interobserver Agreement: The Kappa Statistic. *Family Medicine,* 4